# EUROPEAN PATENT SPECIFICATION

(11) **EP 3 525 802 B1**
(45) Date of publication and mention of the grant of the patent: **26.05.2021**
(21) Application number: 17800641.7
(22) Date of filing: 12.10.2017
(51) Int. Cl.: A61K 35/13, A61P 35/00

(54) **CANCER STEM CELL EXOSOMES**
KREBSSTAMMZELLENEXOSOMEN
EXOSOMES DE CELLULES SOUCHES CANCÉREUSES

(30) Priority: 13.10.2016 US 201662407835 P
(43) Date of publication of application: 21.08.2019
(73) Proprietor: VBC Holdings LLC, Culver City, CA 90232 (US); Niazi, Kayvan, Culver City, CA 90232 (US)
(72) Inventor: NIAZI, Kayvan, Culver City California 90232 (US); CURCIO, Francesco, 33100 Udine (UD) (IT)
(74) Representative: Tomkins & Co
(86) International application number: PCT/US2017/056355
(87) International publication number: WO 2018/071681

(56) References cited:
- WO-A2-01/82958
- US-A1- 2004 028 692
- US-A1- 2006 116 321
- HONG BOK SIL ET AL: "Colorectal cancer cell-derived microvesicles are enriched in cell cycle-related mRNAs that promote proliferation of endothelial cells", BMC GENOMICS, BIOMED CENTRAL, vol. 10, no. 1, 25 November 2009 (2009-11-25), page 556, XP021062243, ISSN: 1471-2164, DOI: 10.1186/1471-2164-10-556
- FERGUSON SCOTT W ET AL: "Exosomes as therapeutics: The implications of molecular composition and exosomal heterogeneity", JOURNAL OF CONTROLLED RELEASE, ELSEVIER, AMSTERDAM, NL, vol. 228, 2 March 2016 (2016-03-02), pages 179-190, XP029495387, ISSN: 0168-3659, DOI: 10.1016/J.JCONREL.2016.02.037

## Description

### TECHNICAL FIELD

The present disclosure relates generally to inactivated exosomes isolated from cancer stem cells, and methods of obtaining or producing the same.

### BACKGROUND OF THE INVENTION

The background description includes information that may be useful in understanding the present invention. It is not an admission that any of the information provided herein is prior art or relevant to the presently claimed invention, or that any publication specifically or implicitly referenced is prior art.

Exosomes are small membrane-bound particles secreted by most cell types, including stem cells, in organisms across a wide taxonomic range (Yu et al., 2014, Int J Mol Sci.7;15(3):4142-57. doi: 10.3390/ijms15034142). Exosomes originate from internal budding of the cellular plasma membrane during endocytotic internalization, from cellular structures identified as multivesicular endosomes (MVE), that package cytoplasmic materials as membrane-bound vesicles. Exosomes have been variously reported to range in diameter from as broadly as from 30nm to about 200 nm, to more particularly from about 40nm to about 100nm. Exosomes have been found to facilitate the delivery and the transfer of proteins, lipids and nucleic acids between cells. Exosomes are released from both normal and diseased cells, and are found in blood and other bodily fluids.

Exosomes have previously been shown to mediate both immunostimulatory and immunoinhibitory modulation of the immune system, e.g., by Zitvogel et al., US20040028692, Whiteside et al. 2005, British Journal of Cancer 92: 209-211. In addition, Robbins et al., US20060116321, describe the immune inhibiting properties of exosomes derived from dendritic cells.

More recently, it has been shown that cancer-derived exosomes have a role in the formation of the tumor microenvironment and progression of tumors (Falcon et al., 2015, J Exp Clin Cancer Res. 34(1): 32, published online 2015 Apr 2. doi: 10.1186/s13046-015-0148-3). Cancer-derived exosomes also help to initiate the inflammatory response, play a role in the differentiation of fibroblasts and mesenchymal cells into myofibroblasts and trigger an angiogenic process. Cancer-derived exosomes also enhance the metastatic evolution of a tumor by promoting epithelial to mesenchymal transformation of tumor cells, and by preparing the tumor niche in a new anatomical location.

In addition, it has been shown that colorectal cancer cell (CRC) - derived exosomes, act to transfer mRNAs from mutant-KRAS and ΔNp73 cells, thus enhancing the invasiveness, proliferation and therapy resistance of recipient CRC cells. SW480 CRC cell line-derived microvesicles are enriched in cell cycle-related mRNAs that promote proliferation of endothelial cells (Hong et al., 2009, BMC Genomics 10:556, DOI: 10.1186/1471-2164-10-556). Further, the expression level of exosomal miR-17-92a cluster has been correlated with the recurrence of CRC (Matsumura et al, 2015, Br J Cancer. 2015, 113(2):275-81. doi: 10.1038/bjc.2015.201. Epub 2015 Jun 9).

The removal of tumor promoting exosomes by extracorporal filtration onto antibody coated microbeads is descrubed by Ichim et al., US8,288,172. However, there remains a need for more practical and effective methods for countering the cancer promoting influence of exosomes derived from cancer stem cells.

### SUMMARY OF THE INVENTION

Accordingly, the invention provides inactivated CSC exosomes and methods of making and using the same to inhibit the cancer promoting effects of CSC derived exosomes. The inventive subject matter is directed to various compositions, methods, and uses of exosomes that are isolated from cancer stem cells that have been exposed to inflammatory conditions.

The invention provides a process for preparing inactivated exosomes comprising the steps of:
(a) isolating cancer stem cells (CSCs) from human cancer tissue,
(b) contacting the isolated CSCs of (a), in a culture medium, with pro-inflammatory cytokines, at a concentration, and for a time period, sufficient to induce release of inflammation induced exosomes,
(c) isolating inflammation induced exosomes from the culture medium of (b), and
(d) inactivating the isolated exosomes of (c) while preserving the membrane structure of the isolated exosomes.

Preferably, the pro-inflammatory cytokines are interferon-gamma (IFNγ), interleukin-la (IL-1a), interleukin-1-β (1IL-1β, interleukin-6 (IL-6), tumor necrosis factor-a (TNFα) and/or combinations thereof. In certain embodiments, the at least one cytokine is present in a concentration ranging from about 10ng/ml to about 50ng/ml.

Generally, the time period in pro-inflammatory culture ranges from about 1 day to about 8 days, or more particularly from about 1 day to about 4 days.

The isolated exosomes are inactivated, e.g., by exposure to ultraviolet radiation (UV) at 254nm and for a duration and intensity effective to inactivate the exosomes without damaging the exosome envelope. The UV exposure is sufficient to inactivate encapsulated RNA molecules and optionally to denature or crosslink encapsulated proteins, e.g., with exposure for about one hour.

In certain embodiments, the CSC are colon cancer stem (CCCS) cells.

The exosomes are preferably isolated from the culture medium by a method such as polymer precipitation, immunological separation, magnetic immunocapture, ultracentrifugation, density gradient centrifugation, size exclusion chromatography, ultrafiltration, ultracentrifugation, density gradient centrifugation, size exclusion chromatography, ultrafiltration and/or combinations thereof.

The invention provides for isolated and purified exosomes produced by the above process.

The invention provides for a pharmaceutical composition comprising the inventive inactivated exosomes, suspended in a physiologically acceptable carrier.

The invention provides a method of inhibiting growth or progression of a tumor or cancer in a subject in need thereof, comprising administering to the subject an effective amount of the inventive inactivated exosomes.

The exosomes are preferably administered by intravenous injection, intramuscular injection, subcutaneous inection, intrathecal injection or infusion and/or intra-organ infusion. For example, the exosomes are administered systemically, in an amount ranging from about 1.5x10¹⁰ to about 1.5x10¹³ exosome particles per kilogram of total body weight. In an alternative example, the exosomes are administered in an amount ranging from about 1.5 x 10¹⁰ and 1.5 x 10¹¹ exosome particles injected or infused into a localized tissue or anatomical space. In certain other embodiments, the invention further includes co-treating the subject with at least one additional anti-cancer agent.

Also disclosed herein is a process for preparing inactivated exosomes comprising the steps of:
(a) isolating cancer stem cells (CSCs) from human cancer tissue,
(b) contacting the isolated CSCs of (a), in a culture medium, with pro-inflammatory cytokines, at a concentration, and for a time period, sufficient to induce release of inflammation induced exosomes,
(c) modifying the isolated CSCs of (a) or (b) to suppress transcription or translation of a gene or genes encoding a cancer promoting RNA or polypeptide, and
(d) isolating inflammation induced exosomes from the culture medium of (b).

### DETAILED DESCRIPTION OF THE INVENTION

The present invention provides exosomes and methods of countering the cancer promoting properties of exosomes derived from cancer stem cells (CSCs).

Unless otherwise indicated, the terms listed below will be used and are intended to be defined as stated, unless otherwise indicated. Definitions for other terms can occur throughout the specification. It is intended that all singular terms also encompass the plural, active tense and past tense forms of a term, unless otherwise indicated.

As understood in the art, the terms "tumor" and "cancer" are overlapping terms. A "tumor" is broadly considered to be a mass or growth found in an organism. A tumor cell is a cell derived from such a mass. A tumor can be benign or cancerous. A cancerous tumor, or "cancer" is a tissue growth that can spread out of control and invade other tissues, or in the case of blood cancers, overwhelm the circulatory system and/or seed cancers elsewhere in the body. A cancer cell is a cell derived from a cancer. For purposes of the invention, the terms "tumor cell" and "cancer cell" are used interchangably, with the understanding that both refer to mammalian cells found in tumors or cancers or derived from and cultured from tumors or cancers, and that replicate abnormally, without the limits exhibited by differentiated mammalian cells.

Exosomes according to the invention are exosomes secreted by tumor cell stem cells, also referred to herein as cancer cell stem cells. The term, "cancer stem cell," or CSC, as used herein, is intended to include cells derived from cancerous tissues of a subject, that have stem cell-like properties, and represent a subset of cancer cells that have the ability to self-renew.

Typically, exosomes secreted by CSCs are particles that, when contacted with normal cells or tissues, will promote the development of tumors, downregulate antitumor immune responses, and/or modify the local tissue to be receptive to establishing metastatic offshoots of an already established tumor or cancer.

The phrase, "inflammatory conditions" means conditions that promote inflammation. For tissues *in vivo,* pro-inflammatory conditions include conditions that promote elevated levels of pro-inflammatory cytokines. For example, pro-inflammatory cytokines include, interferon-gamma, interleukin-la, interleukin-1-β, interleukin-6, tumor necrosis factor-a, and combinations thereof.

Such conditions include, for example, conditions that require a healing or anti-infective response, such as physical injury, infection or chronic irritation of tissue caused by metabolic dysfunction or infiltration of foreign materials into the tissue. For tissue or cells *in vitro,* pro-inflammatory conditions include culturing cells or tissues in the presence of pro-inflammatory cytokines or co-culturing cells or tissues with cells or tissues that secrete pro-inflammatory cytokines.

For the present invention, such conditions also include the presence of a diagnosed existing tumor or cancer condition, wherein the inventive inactivated exosomes are administered to inhibit the formation and spread of such existing tumor or cancer condition.

The term "culturing" refers to the *in vitro* maintenance, differentiation, and/or propagation of cells in suitable media. When referring to an "enriched" culture, it is meant a composition comprising components, e.g., cells, present in a greater percentage of total cells than is found in the tissues where they are present in an organism.

The phrase "consisting essentially of" means that the composition or method may include additional ingredients and/or steps, but only if the additional ingredients and/or steps do not materially alter the basic and novel characteristics of the claimed composition or method, i.e., the additional ingredient and/or step(s) would serve no purpose material to the claimed composition or method.

Preferably, the subject that is the source of the CSC is an animal such as a mammal or an avian. Animals include humans, and non-human veterinary subjects. Veterinary subjects include both mammals and avians. Mammals, include, without limitation, domestic dogs, and other canines, domestic cats, and other felines, pigs and other porcines, sheep, goats, horses and other equines, camels, cattle and other ungulates. An avian is contemplated to include fowl, e.g., chickens, ducks, turkeys, geese, ostrich and the like, and/or pet avians, such as finches and/or members of the order of *Psittaciformes*, e.g., parrots and parakeets

Methods for isolating CSCs are known to the art. For example, in one method, cancer tissue is disaggregated, digested enzymatically, washed and filtered. The harvested cells are then cultured in a medium that allows the cells to form spheres. *See,* for example, Pramudita, et al., 2013, Methods Mol Biol, 1035: 247-59, doi: 10.1007/978-1-62703-508-8_21 (published as Chapter 21 in STEM CELL NICHE METHODS AND PROTOCOLS, by Springer, Ed.: Turksen, Kursad). *See* also, Yamazaki et al., US 20140314675, and Yu, US20100173344 describing additional approaches to isolating CSCs.

Isolated CSCs are cultured and maintained in a suitable medium, e.g., as described by Pramudito, Id.

In a preferred embodiment of the invention, the isolated CSCs are cultured in a medium supplemented with one or more pro-inflammatory cytokines such as interferon-gamma (IFNγ), interleukin-la (IL-1α), interleukin-1-β (1IL-1β), interleukin-6 (IL-6), tumor necrosis factor-a (TNFα) and combinations thereof. The cytokine or cytokines are present in a concentration, and for a time period, sufficient to induce the release of inflammation related exosomes form the CSC. For example, the, one or more cytokines are present in a concentration ranging from about 10ng/ml to about 50ng/ml. Generally, the CSC are cultured for from about 1 day to about 8 days, or more. Alternatively, the CSC are cultured for from about 1 day to about 4 days, or more.

After incubating cultured CSC a sufficient time period to accumulated CSC exosomes, e.g., for from about 2 to about 4 days at 37° C, the culture medium is collected and the exosomes purified and isolated from the culture medium. This can be accomplished by any suitable art-known method. For example, see Robbins et al., US20060116321, or Lane et al., Id., Brownlee, et al., 2014, J Immunol Methods, 407: 120-126. doi: 10.1016/j.jim.2014.04.003. These methods include, for example, the original method of separating exosomes by differential ultracentrifugation, and newer methods, such as polymer precipitation (ExoQuick™ from SBI, Palo Alto, CA), immunoaffinity capture (Greening et al. 2015, Methods in Molecular Biology, Impact Factor: 1.29), immune magnetic capture (Exo-FLOW™, SBI), the Invitrogen Total Exosome Isolation Kit (Life Technologies, USA) and the ExoSpin Exosome Purification Kit (Cell Guidance Systems, USA).

Immuno-affinity purification is a method to selectively capture specific exosomes based upon surface markers. This approach employs magnetic beads covalently coated with streptavidin, which can be coupled in high affinity fashion with biotinylated capture antibody. Captured exosomes are eluted and are intact and bioactive.

Purified exosomes are quantified by determining the protein content and the activity of acetyl-CoA acetylcholinesterase, and are analyzed for size distribution and concentration by nanoparticle tracking analysis. The isolated exosomes are validated for exosomal marker expression by flow cytometry and Western blot.

The isolated exosomes are then inactivated without damaging the membrane structure of the exosomes. This can be accomplished by exposing the isolated exosomes to conditions known to cause breaks or crosslinking in the protein and/or RNA molecules packaged by the exosomes. For example, the exosomes are exposed to ultraviolet radiation at a frequency, intensity and duration sufficient to inactivate RNA molecules carried by the isolated exosomes without disrupting the exosome surface proteins. See, e.g., Pusic et al. WO2014028763. Alternatively, the isolated CSC are genetically modified to suppress production of cancer promoting RNA or proteins, so that the produced exosomes lack the activity of promoting cancer progression. In another alternative, artificial exosomes with envelope proteins matching the cell surface antigenic epitopes of CSCs are prepared, wherein the artificial exosomes do not carry cancer promoting proteins and/or cancer promoting RNA molecules.

The invention also provides methods of treating subjects, including mammalian subjects. In particular, the invention provides methods for inhibiting or reducing the spread of a tumor or cancer condition in a subject that has been diagnosed and/or treated for such a tumor or cancer condition.

Without meaning to be bound by any theory or hypothesis as to the operation of the invention, it is believed that the inventive inactivated exosomes compete with unmodified natural CSC exosomes, produced *in vivo,* for update by target cells and tissues. In this way, the cancer promoting effects of the naturally produced CSC exosomes are inhibited.

For example, the method includes administering the inventive inactivated exosomes to a subject who has been diagnosed with a cancer, wherein the exosomes are administered systemically, and/or infused into a tissue or anatomical space, in an amount, and for a duration, sufficient to inhibit the malignant spread, or the further malignant spread, of the original cancer.

An "effective amount" is an amount sufficient to effect beneficial or desired results, such as a reduced rate for the progression of a tumor or cancer. An effective amount can be administered in one or more administrations, applications or dosages. The effective amount, i.e., a suitable dosage, will vary depending on body weight, age, health, disease or condition to be treated and route of administration. The dose of exosomes administered to a subject is in an amount effective to achieve the desired beneficial therapeutic response in the subject over time.

The artisan will be readily able to determine the amount of inactivated exosomes to be administered by titrating the dose and duration of administration to reach an optimal clinical response, such as a reduction in the rate of progresson and/or spread of a cancer, and/or inducing the regression of the cancer.

In a particular embodiment, the inactivated exosomes are administered systemically, in an amount ranging from about 1.5 x 10¹⁰ to about 1.5 x 10¹³ exosome particles per kilogram of total body weight. Aternatively, the inactivated exosomes are administered in an amount ranging from about 1.5 x 10¹⁰ and 1.5 x 10¹¹ exosome particles injected or infused into a localized tissue or anatomical space.

The inactivated exosomes are optionally administered by a route selected from the group consisting of, intravenous injection, intramuscular injection, subcutaneous inection, intrathecal injection or infusion and intraorgan infusion. Intraorgan infusion includes, infusion into anatomical spaces, such as, simply by way of example, the gallbladder, gastrointestinal lumen, esophagous, pulmonary system (by inhalation) and/or urinary bladder.

The number of inactivated exosomes in a preparation can be determined by any art known method. In a non-limiting example, exosome particle numbers can be determined by direct counting using a NanoSight instrument, such as a NanoSight® NS300, NanoSight NS500® or NanoSight® LM10 (Malvern Instruments, Ltd, Worcestershire, UK). Alternatively, the number of exosomes can be estimated by measuring the activity of acetyl-CoA acetylcholinesterase, an enzyme present within exosomes, and then estimating the exosome count by reference to a pre-prepared standard curve of exosome counts verses Acetyl Co-A levels.

The treatment is repeated as needed until a positive result is obtained. Optionally, the treatment is repeated at a daily, weekly or monthly interval, as needed, in order to maintain suppression of the cancer promoting process.

In a further embodiment, the invention contemplates co-treating a subject in need thereof, with at least one additional anti-cancer agent, such as, alkylating agents including platinum compounds such as cisplatin, carboplatin and/or oxaliplatin; antimetabolites, e.g., methotrexate, 5-fluorouracil, and/or cytarabibe, agents derived from natural products, e.g. L-asparaginase, doxorubicin, bleomycin, taxanes such as paclitaxel, docetaxel epipodophyllotoxins: etoposide, camptothecins and/or irinotecan.

In particular, the at least one additional anti-cancer agent includes, but is not limited to, docetaxel, gemcitabine, imatinib (Gleevec®), 5-fluorouracil, 9-aminocamptothecin, amine-modified geldanamycin, doxorubicin, paclitaxel (Taxol®), cisplatin, procarbazine, hydroxyurea, meso e-chlorin, Gd(+3) compounds, asparaginase, and/or radionuclides (e.g., I¹³¹, Y⁹⁰, In¹¹¹, and Tc-99m). In certain embodiments, two or more anticancer agents are administered together with the inventive inactivated exosomes.

In particular, the at least one additional anti-cancer agent includes, but is not limited to, docetaxel, gemcitabine, imatinib (Gleevec®), 5-fluorouracil, 9-aminocamptothecin, amine-modified geldanamycin, doxorubicin, paclitaxel (Taxol®), cisplatin, procarbazine, hydroxyurea, meso e-chlorin, Gd(+3) compounds, asparaginase, and radionuclides (e.g I¹³¹, Y⁹⁰, In¹¹¹, and Tc-99m). Some embodiments include two or more supplementary anticancer agents. In certain embodiments, two or more anticancer agents are administered together with the inventive inactivated exosomes.

### EXAMPLES

The following examples are provided in order to illustrate the present invention, without intending to limit the scope of the present invention.

### EXAMPLE 1

### ISOLATING COLON CANCER STEM CELLS (CCSCs)

Isolated colon cancer stem cells or CCSC are obtained by isolating the cells from tissue samples obtained from a subject with colon cancer. The subject can be a human or an animal, including, for example, a mouse with xenografted tumors grown from colon cancer cells that originated in a human or non-human cancer cell line.

The tissue samples are then processed essentially as described by Pramudita, et al., 2013, Methods Mol Biol, 1035: 247-59, doi: 10.1007/978-1-62703-508-8_21 (published as Chapter 21 in STEM CELL NICHE METHODS AND PROTOCOLS, by Springer, Editors: Turksen, Kursad; and employing the methods and reagents described therein for isolating both colon cancer stem cells and adenoma stem cells).

The resected cancerous tissue samples are handled under aseptic conditions while following biological safety level 2 protocols, by the method as described by Pramudita et al. at Section 3.1 of that publication. The Pramudita method for isolating colon cancer stem cells is applied herein, as summarized, in brief, as follows.

The tissue samples are placed directly into one or more tubes of appropriate size, containing Hank's Balanced Salt Solution 1× (HBSS) without Ca 2+ and Mg 2+ (Invitrogen).

The tissue samples are then washed to remove blood and surgical debris. Excess necrotic tissue is removed from the sample, followed by further washing with HBSS. The washed samples are then minced, and the minced tissue digested at 37°C for 30-60 min, with occasional agitation in culture medium containing collagenase type IV (Sigma, 100 mg/ml in phosphate buffer [PBS] with a 1 part in 100 working dilution); and hyaluronidase type IV-S (Sigma 10 mg/ml in PBS, with a 1 part in 500 working dilution).

The digested tissue and culture medium is centrifuged, and the supernatant containing single cells is recovered, recentrifuged, washed, screened (70 µm mesh) and washed as needed to remove undigested fragments and digestion enzymes. The remaining washed cell pellets are resuspended and the resuspended viable single cells are counted.

From 5 x 10⁴ to 10 x 10⁴ cells/cm² are seeded into growth factor supplemented culture medium DMEM F12 Advanced medium supplemented with N2 supplement 100x, Glucose 8.5mM, Trace Element B and C 1000x, Hepes 50µM, Heparine 2µg/ml, Insuline 10µg/ml, β-Mercaptoethanol 0.1mM, L-Glutamine 0.4mM, EGF 20ng/ml and bFGF 10ng/ml] in ultralow adherent vessels, where the CCSC cells should self-organize into small spheroid structures. The culture is passaged as needed, as described by Pramudita et al. at Section 3.2 of that publication, to maintain viability.

### EXAMPLE 2

### CULTURING ISOLATED CSC UNDER INFLAMMATORY CONDITIONS

The CCSC isolated according to Example 1 are continued in the same culture medium, or are passaged into fresh culture medium, according to Pramudita et al. The culture medium is supplemented with one or more pro-inflammatory cytokines, such as, one or more of interferon-gamma (IFNγ), interleukin-la (IL-1a), interleukin-1-β (1IL-1β), interleukin-6 (IL-6), tumor necrosis factor-a (TNFα). Each pro-inflammatory cytokine is added to the culture medium in a concentration ranging from about 10ng/ml to about 50ng/ml,

### EXAMPLE 3

### ISOLATION OF EXOSOMES FROM COLON CANCER STEM CELLS

Exosomes are isolated from the culture medium employed by Example 2, after the CSC were under culture for from 1 to 8 days. The exosomes are isolated by the polymer precipitation (ExoQuick™ from SBI, Palo Alto, CA). This technology captures and collects exosomes in "polymer nets," that are recovered by a low speed centrifugation. Once the exosome pellet was obtained, the supernatant containing excess polymer was removed and the pelleted exosomes were then resuspended in phosphate-buffered saline (PBS) solution, dissolving the polymer net and releasing intact exosomes.

### EXAMPLE 4

### VALIDATING ISOLATED CCSC EXOSOMES

In order to confirm that functional exosomes are isolated by the polymer precipitation process of Example 3, the identity of the exosomes is validated by measuring the presence of appropriate biomarkers, as follows.

Exosomes are isolated by the method described above, and the exosomes validated by Western blot, using antibodies targeting specific exosomal markers: tetraspanins, CD9, CD63, CD81 (localized to the exosomal surface) and the protein expressed by tumor susceptibility gene 101 ("TSG101") that is found inside exosomes.

Isolated exosomes are lysed in RIPA buffer (150 mM sodium chloride, 1% NP-40, 0.5% sodium deoxycholate, 0.1% SDS, 50 mM Tris, pH 8.0) supplemented with protease inhibitors (Sigma-Aldrich) for 30 minutes on ice. The lysate is quantified for Bradford assay (Bio-Rad) and 25µg of proteins are mixed with 4x sample buffer (8% SDS, 20% 2-mercaptoethanol, 40% glycerol, 0.008% bromophenol blue, 0.25M Tris, pH 6.8) and boiled for 10 minutes at 95°C. Proteins are resolved by SDS-PAGE, transferred to PVDF membranes, blocked in 5% non-fat powdered milk or BSA in TBS-T (20 mM Tris pH 7.5, 150 mM NaCl, 0.1% Tween-20) and probed with the following anti-human antibodies: anti-CD9 (1:1000, SBI), anti-CD63 (1:1000, LS Bio), anti-CD81 (1:500, Abcam), anti-TSG101 (1:500, Abcam). Secondary antibodies conjugated to horseradish peroxidase (1:1000, Dako) visualize the proteins by way of chemilluminescence (ECL Western blotting substrate, Thermo Scientific).

In addition, in order to further validate the exosomes by flow cytometry, exosomes are bound to latex beads that are conjugated with anti-CD63 antibody, thus avoiding any non-specific binding of the beads that might result from their small size. The expression of CD81 and CD9 by the exosomes is confirmed by cytofluorimetric data, where CD7 is used as a negative control to demonstrate the specificity of the assay. The primary antibodies used are: anti-CD9 Alexa 647 (Serotec), anti-CD81 FITC (Biolegend) and anti-CD7 PE (Becton Dickinson) or isotype control (BD Biosciences). Exosomes are analyzed using a FACSCalibur flow cytometer (BD Biosciences).

The obtained exosomes are quantified by determining the activity of acetyl-CoA esterase ("AChE," Exocet™ test kit, SBI). AChE is an enzyme known to be found within all exosomes tested to date. A standard curve, as measured by NanoSight® analysis, that was calibrated to exosome numbers, is included in the Exocet kit.

### EXAMPLE 5

### INACTIVATION OF ISOLATED CCSC EXOSOMES

Isolated exosomes obtained as above are inactivated without damaging the exosome membranes by rendering the membrane enclosed RNA and/or proteins inactive by the following method.

Isolated exosomes are inactivated by any art known methods. In one embodiment, the exosomes are inactivated by exposure to 254 nm ultraviolet (U.V.) light, as described by WO2014028763, for one hour.

### EXAMPLE 6

### CANCER SUPPRESSING ACTIVITY OF INACTIVATED EXOSOMES

Exosomes obtained by the method of Example 5, from CCSC, are tested for the property of interfering with the maintenance and/or development of a cancer promoting microenvironment is confirmed. This is done by the measuring the rate of progression of seeded human cancer cells in mice infused with inactivated CCSC exosomes, in a mouse xenograft model, relative to the progression of seeded human cancer cells in the same model, treated with a control formulation lacking inactivated exosomes, as follows.

Human HT-29 colorectal tumors are established into 24 nude mice by subcutaneous injection of 1 x 10⁶ cells/mouse into a right auxiliary flank. When tumors reach an average volume of 100 mm³, half of the mice are injected intravenously (once daily for 14 days) with from 1.5 x 10¹⁰ to about 1.5 x 10¹³ inactivated exosome particles per kg/total body weight, suspended in an physiologically acceptable carrier solution, and half are treated with the physiologically acceptable carrier solution without any exosomes as a control. The mice are monitored for tumor growth. The exosomes and control fluids are administered intravenously via the tail vein.

The results of the treated mice compared to the control mice, confirms that the rate of increase in the volume, weight and spread (number of secondary tumors) of the xenografted cancers is significantly reduced in the group of 12 mice injected with the inactivated exosomes, as measured *in vivo* and after dissection of the mouse subjects.

## Claims

1. A process for preparing inactivated exosomes comprising the steps of:
(a) isolating cancer stem cells (CSCs) from human cancer tissue,
(b) contacting the isolated CSCs of (a), in a culture medium, with pro-inflammatory cytokines, at a concentration, and for a time period, sufficient to induce release of inflammation induced exosomes,
(c) isolating inflammation induced exosomes from the culture medium of (b), and
(d) inactivating the isolated exosomes of (c) by exposure to ultraviolet radiation, wherein the ultraviolet radiation is at intensity and frequency sufficient to substantially inactivate RNA contained in the isolated exosomes, while preserving the membrane structure of the isolated exosomes.

2. The process of claim 1, wherein the pro-inflammatory cytokines are selected from the group consisting of interferon-gamma (IFNγ), interleukin-la (IL-1α), interleukin-1-β (IL-1β), interleukin-6 (IL-6), tumor necrosis factor-a (TNFα) and combinations thereof.

3. The process of claim 2, wherein the at least one cytokine is present in a concentration ranging from 10ng/ml to 50ng/ml.

4. The process of claim 1, wherein the time period in pro-inflammatory culture ranges from 1 day to 8 days.

5. The process of claim 4, wherein the time period in pro-inflammatory culture ranges from 1 day to 4 days.

6. The process of claim 1, wherein the CSC are colon cancer stem (CCCs) cells.

7. The process of claim 1, wherein the exosomes are isolated from the culture medium by a method selected from the group consisting of polymer precipitation, immunological separation, magnetic immunocapture, ultracentrifugation, density gradient centrifugation, size exclusion chromatography, ultrafiltration and combinations thereof.

8. Isolated and purified exosomes produced by the process of claim 1.

9. A pharmaceutical composition comprising the exosomes of claim 8, and a physiologically acceptable carrier.

10. Exosomes of claim 8 for use in inhibiting growth or progression of a tumor or cancer.

11. Exosomes for use according to claim 10, wherein the tumor or cancer is a tumor or cancer of a mammal selected from the group consisting of a human, a canine, a feline, a porcine and an equine.

12. Exosomes for use according to claim 11, wherein the exosomes are administerable by a route selected from the group consisting of, intravenous injection, intramuscular injection, subcutaneous inection, intrathecal injection or infusion and intraorgan infusion.

13. Exosomes for use according to claim 12, wherein the exosomes are administerable systemically, in an amount ranging from 1.5 x 10¹⁰ to 1.5 x 10¹³ exosome particles per kilogram of total body weight.

14. Exosomes for use according to claim 13, wherein exosomes are administerable in an amount ranging from 1.5x10¹⁰ to 1.5 x 10¹¹ exosome particles injected or infused into a localized tissue or anatomical space.

15. Exosomes for use according to claim 13, further comprising use of at least one additional anti-cancer agent, and/or combinations thereof.

## Patentansprüche

1. Verfahren zur Herstellung von inaktivierten Exosomen, umfassend die Schritte:
(a) Isolieren von Krebsstammzellen (cancer stem cells; CSCs) aus humanem Krebsgewebe,
(b) Inkontaktbringen der isolierten CSCs aus (a) mit pro-inflammatorischen Cytokinen in einem Kulturmedium bei einer Konzentration und über einen Zeitraum, ausreichend, um die Freisetzung von Entzündungs-induzierten Exosomen zu induzieren,
(c) Isolieren der Entzündungs-induzierten Exosome aus dem Kulturmedium aus (b), und
(d) Inaktivieren der isolierten Exosome aus (c) durch Aussetzen gegenüber Ultraviolettstrahlung, wobei die Ultraviolettstrahlung mit einer Intensität und Frequenz erfolgt, ausreichend, um in den isolierten Exosomen enthaltene RNA wesentlich zu inaktivieren, während die Membranstruktur der isolierten Exosomen aufrechterhalten wird.

2. Verfahren nach Anspruch 1, wobei die pro-inflammatorischen Cytokine ausgewählt sind aus der Gruppe bestehend aus Interferon-Gamma (IFNγ), Interleukin-1-α (IL-1α), Interleukin-1-β (IL-1β), Interleukin-6 (IL-6), Tumornekrosefaktor-a (TNFα) und Kombinationen davon.

3. Verfahren nach Anspruch 2, wobei das mindestens eine Cytokin in einer Konzentration in einem Bereich von 10 ng/ml bis 50 ng/ml vorliegt.

4. Verfahren nach Anspruch 1, wobei der Zeitraum in pro-inflammatorischer Kultur in einem Bereich von 1 Tag bis 8 Tagen liegt.

5. Verfahren nach Anspruch 4, wobei der Zeitraum in pro-inflammatorischer Kultur in einem Bereich von 1 Tag bis 4 Tagen liegt.

6. Verfahren nach Anspruch 1, wobei die CSC Krebsstammzellen des Kolons (colon cancer stem cells; CCCs) sind.

7. Verfahren nach Anspruch 1, wobei die Exosome aus dem Kulturmedium durch ein Verfahren isoliert werden, das ausgewählt ist aus der Gruppe bestehend aus Polymerpräzipitation, immunologischer Trennung, magnetischem Immunocapture, Ultrazentrifugation, Dichtegradientenzentrifugation, Größenausschlusschromatographie, Ultrafiltration und Kombinationen davon.

8. Isolierte und gereinigte Exosome, wie durch das Verfahren nach Anspruch 1 hergestellt.

9. Arzneimittel, das die Exosome nach Anspruch 8 und einen physiologisch verträglichen Träger umfasst.

10. Exosome nach Anspruch 8 zur Verwendung bei der Inhibierung des Wachstums oder des Fortschreitens eines Tumors oder einer Krebserkrankung.

11. Exosome zur Verwendung nach Anspruch 10, wobei der Tumor oder die Krebserkrankung ein Tumor oder eine Krebserkrankung eines Säugers ist, ausgewählt aus der Gruppe bestehend aus einem Menschen, Canidae, Felidae, Suidae und Equidae.

12. Exosome zur Verwendung nach Anspruch 11, wobei die Exosome über einen Weg verabreichbar sind, der ausgewählt ist aus der Gruppe bestehend aus intravenöser Injektion, intramuskulärer Injektion, subkutaner Injektion, intrathekaler Injektion oder Infusion und intraorganer Infusion.

13. Exosome zur Verwendung nach Anspruch 12, wobei die Exosome in einer Menge im Bereich von 1,5 x 10¹⁰ bis 1,5 x 10¹³ Exosompartikel pro Kilogramm des gesamten Körpergewichts systemisch verabreichbar sind.

14. Exosome zur Verwendung nach Anspruch 13, wobei die Exosome in einer Menge im Bereich von 1,5 x 10¹⁰ bis 1,5 x 10¹¹ Exosompartikel durch Injektion oder Infusion in ein lokalisiertes Gewebe oder anatomischen Raum verabreichbar sind.

15. Exosome zur Verwendung nach Anspruch 13, des Weiteren umfassend die Verwendung von mindestens einem zusätzlichen Anti-Krebsmittel und/oder Kombinationen davon.

## Revendications

1. Procédé pour préparer des exosomes inactivées, comprenant les étapes :
(a) isoler des cellules souches cancéreuses (CSC) à partir d'un tissu cancéreux humain,
(b) mettre en contact les CSC isolées de (a), dans un milieu de culture, avec des cytokines pro-inflammatoires, à une concentration et pendant une période suffisante pour induire la libération d'exosomes induits par l'inflammation,
(c) isoler les exosomes induites par l'inflammation à partir du milieu de culture de (b), et
(d) inactiver les exosomes isolées de (c) par une exposition à un rayonnement ultraviolet, où le rayonnement ultraviolet est à une intensité et une fréquence suffisantes pour essentiellement inactiver l'ARN contenu dans les exosomes isolés, tout en préservant la structure membranaire des exosomes isolés.

2. Procédé selon la revendication 1, dans lequel les cytokines pro-inflammatoires sont sélectionnées dans le groupe consistant en l'interféron-gamma (IFNγ), l'interleukine-1α (IL-1α), l'interleukine-1β (IL-1β), l'interleukine-6 (IL-6), le facteur de nécrose tumorale α (TNFα) et des combinaisons de ceux-ci.

3. Procédé selon la revendication 2, dans lequel la au moins une cytokine est présente en une concentration comprise entre 10 ng/ml et 50 ng/ml.

4. Procédé selon la revendication 1, dans lequel la période de culture pro-inflammatoire est comprise entre 1 jour et 8 jours.

5. Procédé selon la revendication 4, dans lequel la période de culture pro-inflammatoire est comprise entre 1 jour et 4 jours.

6. Procédé selon la revendication 1, dans lequel les CSC sont des cellules souches du cancer du côlon (CCCs).

7. Procédé selon la revendication 1, dans lequel les exosomes sont isolés à partir du milieu de culture par un procédé sélectionné dans le groupe consistant en une précipitation polymère, une séparation immunologique, une capture immunomagnétique, une ultracentrifugation, une centrifugation en gradient de densité, une chromatographie d'exclusion stérique, une ultrafiltration, une ultracentrifugation, une centrifugation en gradient de densité, une chromatographie d'exclusion stérique, une ultrafiltration et des combinaisons de ceux-ci.

8. Exosomes isolés et purifiés produits par le procédé selon la revendication 1.

9. Composition pharmaceutique comprenant les exosomes selon la revendication 8, et un véhicule physiologiquement acceptable.

10. Exosomes selon la revendication 8 pour utilisation dans l'inhibition de la croissance ou de la progression d'une tumeur ou d'un cancer.

11. Exosomes pour utilisation selon la revendication 10, où la tumeur ou le cancer est une tumeur ou un cancer d'un mammifère sélectionné dans le groupe consistant en un humain, un canidé, un félin, un porcin et un équidé.

12. Exosomes pour utilisation selon la revendication 11, où les exosomes peuvent être administrés par une voie sélectionnée dans le groupe consistant en une injection intraveineuse, une injection intramusculaire, une injection sous-cutanée, une injection intrathécale ou une perfusion et une perfusion intra-organe.

13. Exosomes pour utilisation selon la revendication 12, où les exosomes peuvent être administrés par voie systémique, en une quantité comprise entre 1,5 x 10¹⁰ et 1,5 x 10¹³ particules d'exosomes par kilogramme de poids corporel total.

14. Exosomes pour utilisation selon la revendication 13, où les exosomes peuvent être administrés en une quantité comprise entre 1,5 x 10¹⁰ et 1,5 x 10¹¹ particules d'exosomes injectées ou perfusées dans un tissu localisé ou un espace anatomique.

15. Exosomes pour utilisation selon la revendication 13, comprenant en outre l'utilisation d'au moins un agent anticancéreux supplémentaire, et/ou de combinaisons de celui-ci.
